# EUROPEAN PATENT APPLICATION

(11) **EP 4 289 868 A1**
(43) Date of publication of application: **13.12.2023**
(21) Application number: 22758916.5
(22) Date of filing: 24.02.2022
(51) Int. Cl.: C07K 19/00, C12N 15/09, A61K 38/16, A61P 25/00

(54) **MODIFIED NEUROTOXIN SINGLE-CHAIN POLYPEPTIDE AND USE THEREOF**

(30) Priority: 26.02.2021 CN 202110217647
(71) Applicant: Chongqing Claruvis Pharmaceutical Co., Ltd., Chongqing 400713 (CN)
(72) Inventor: ZHANG, Yan, Chongqing 400722 (CN)
(74) Representative: HGF
(86) International application number: PCT/CN2022/077631
(87) International publication number: WO 2022/179552

(57) **Abstract**

The present invention relates to a modified neurotoxin single-chain polypeptide and a use thereof. The single-chain polypeptide contains a tagged protein, a linker short peptide, etc. The linker short peptide facilitates cleavage of the tagged protein, thereby improving purification of the single-chain polypeptide; in addition, the single-chain polypeptide has neurotoxicity when activated, but the single-chain polypeptide per se is only slightly toxic, such that the production safety is improved and the production process cost is reduced while the yield of the single-chain polypeptide is greatly increased.

## Description

### PRIORITY

The present application requires priority to Application No. 202110217647.X, filed Feb. 26, 2021, which is incorporated herein in its entirety by reference.

### TECHNICAL FIELD

The present invention belongs to the technical field of biology, and particularly relates to a modified single-chain polypeptide of a neurotoxin and use thereof.

### BACKGROUND

Neurotoxins, including, for example, botulinum neurotoxins (BoNTs for short) and tetanus toxins (*clostridium tetani,* tetX or TeNT for short), both of which belong to clostridial neurotoxins (CNTs), are typical highly potent neurotoxins, with lethal doses in humans of between 0.1 ng and 1 ng per kilogram of body weight (Tonello et al., Adv. Exp. Med. & Biol. 389: 251-260 (1996)). Clostridial neurotoxins are capable of specifically binding to nerve cells and disrupting neurotransmitter release.

*Clostridium botulinum* secretes 7 serotypes having different antigens, and these serotypes are designed as botulinum neurotoxins (BoNTs) type A to G. Although botulinum neurotoxins (BoNTs) only have 65% homology to tetanus toxins (tetX), they have almost identical molecular structures and molecular mass, and both comprise two disulfide-linked amino acid chains: a light chain (L) of about 50 KDa and a heavy chain (H) of about 100 KDa. The light chain comprises a protease activity domain and the heavy chain comprises a translocation domain (N-terminal) and a receptor-binding domain (C-terminal). A holotoxins is synthesized *in vivo* as a single chain, and a single-chain polypeptide comprising a heavy chain is subsequently cleaved in a post-translational modification, the process of which involves proteolysis of an exposed region called a loop region by protein, eventually forming an active neurotoxin comprising an L chain and an H chain, which are linked by a disulfide bond. All serotypes of botulinum neurotoxins (BoNTs) and related tetanus neurotoxins (TexT) secreted by *clostridium tetani* are Zn²⁺ proteases that prevent synaptic exocytosis, inhibit neurotransmitter release and interrupt nerve signaling by cleaving a protein involved in the formation of an SNARE complex that controls cell membrane fusion. The botulinum neurotoxin inhibits the release of peripheral excitatory neurotransmitters, causing muscle paralysis, while tetanus neurotoxin inhibits the release of central inhibitory neurotransmitters, causing muscle spasm. Furthermore, the CNT activity has been proven to affect secretion of glands.

Botulinum neurotoxin type A (BoNT/A) has been approved for being used in the treatment of strabismus, blepharospasm and other diseases in 1989 in the United States. In some applications, a botulinum neurotoxin is injected directly into a muscle to be treated with an additional bacterial protein in the form of a complex, and the toxin is released from the protein complex (Eisele et al. 2011, Toxicon 57 (4): 555-65) and produces the desired pharmacological effect at a physiological pH value. In other applications, the improved BoNT/A formulation does not contain complex protein. Since the BoNT's effect is only temporary, patients need to take the BoNT repeatedly to maintain therapeutic efficacy. The FDA has further approved botulinum neurotoxin type B for being used in the treatment of cervical dystonia.

Among the various therapeutic applications using the botulinum neurotoxin, U.S. Patent No. 6,113,915 and U.S. Patent No. 5,721,215 disclose use for the treatment of pain; U.S. Patent No. 5,053,005 discloses use of botulinum neurotoxins for the treatment of neuromuscular diseases; Elston, J. S. et al. disclose use of BoNT/A in the treatment of strabismus (British Journal of Ophthalmology, 1985, 69, 718-724 and 891-896); Adenis, J. P. et al. disclose use of BoNT/A in the treatment of blepharospasm (Ophthalmol., 1990, 13(5), 259-264); Jankovic et al. disclose use of BoNT/A in the treatment of spasmodic oromandibular dystonic torticollis (Neurology, 1987, 37, 616-623); Blitzer et al. disclose examples of the treatment of spasmodic dysphonia with BoNT/A (Ann. Otol. Rhino. Laryngol, 1985, 94, 591-594); Brin et al. disclose use of BoNT/A in the treatment of tongue dystonia (Adv. Neurol. (1987)50, 599-608); Cohen et al. disclose use of BoNT/A in the treatment of writer's cramp (Neurology (1987) 37 (Suppl. 1), 123-4).

Despite the clear therapeutic and cosmetic effects of the botulinum neurotoxin, industrial production of the toxin has faced difficulties, which focus on both purity and safety (low or no toxicity).

In terms of purity, in order to obtain recombinant proteins, in particular smaller polypeptides, in sufficient quantities and in a soluble form, in many cases the proteins and polypeptides can be expressed in *Escherichia coli* as fusion or hybrid proteins, such as fusion or hybrid proteins with glutathione-S-transferase or maltose binding protein. Furthermore, there are many expression systems on the market for expressing the desired polypeptide by means of an N-terminal or C-terminal tag for affinity purification, such as a His tag, Strep tag or FLAG tag. However, in the pharmaceutical industry, the subsequent addition of protein has to prove that the protein and its possible contaminants are completely removed in further processing, which usually entails considerable expenditure. Therefore, how to remove the tag protein as much as possible while improving the purity is a demand for the production of industrial polypeptide formulations.

The heavy chain and light chain of the clostridial neurotoxin are linked to each other by a disulfide bond. Among the participating cysteine residues are linker or loop regions (a loop, also defined in the industry as a linker sequence or loop sequence) whose length varies widely among the various serotypes of clostridial neurotoxins. The loop is cleaved by a hitherto uncharacterized clostridial endopeptidase at the latest when the toxin is released from the clostridia during cell lysis, where the ratio of cleaved and uncleaved species varies between serotypes. For example, for BoNT/A, the loop sequence VRGIITSKTKSLDKGYNKALNDL is cleaved by a protease at two enzyme cleavage sites during cleavage and a decapeptide is cut out. The cleaved light chain and heavy chain are disulfide-linked at the N- and C-termini by cysteine residues. However, in this process, the cleavage by the protease (at the ends of the loop region and the heavy chain) is random, thereby producing many undesired intermediates, increasing the difficulty of purification and reducing the yield of the product.

In terms of the safety due to high toxicity, there is a method for expressing and purifying the heavy chain and light chain of TeTx and BoNTs, respectively, in *Escherichia coli* (see Li, et al., Biochemistry 33: 7014-7020 (1994); Zhou, et al., Biochemistry, 34: 15175-15181 (1995)). The isolated chains in *Escherichia coli* are non-toxic by themselves, and then double chains are formed by binding the H and L subunits with oxidative disulfide bonds. However, the method is not practical because, firstly, in the absence of L chains, the isolated H chains are poorly water soluble and are very susceptible to proteolytic degradation, and secondly, the *in vitro* oxidation of separately expressed and purified H and L chains to produce active double chains is inefficient and results in the production of many inactive and incorrectly folded or oxidized molecules, making it very difficult to isolate and purify H and L chains containing correct folding and oxidation therefrom.

It is therefore practical to express neurotoxins as inactive (or less active) single-chain forms, to maintain solubility of the protein chain, to reduce protein misfolding and resulting susceptibility to protease attack, and to improve toxin production. One approach is to modify the site in the cyclic structure of the neurotoxin to the site that is recognized and hydrolyzed only by a specific protease. Patent US7709228 discloses enzyme cleavage sites that can be modified and proteases that can specifically recognize these sites. In the specific embodiment of the patent, the EK enzyme cleavage site is used for modifying the loop region of TeTx, but the toxicity of the recombinant product is equivalent to two-fifths of the natural TeTx, and the reduction is not very significant. The patent also compares the proteolytic activity and toxicity of uncleaved recombinant BoNT/E with natural BoNT/E. Although the uncleaved recombinant BoNT/E is significantly less active, the toxicity thereof is not greatly reduced.

### SUMMARY

In order to solve the problems of thoroughly removing the tag protein while improving the purity, and reducing the toxicity and safe production in the production of recombinant neurotoxin polypeptides, the present invention provides a modified neurotoxic single-chain polypeptide and use thereof, which are specifically as follows.

In a first aspect of the present invention, provides a modified single-chain polypeptide of a neurotoxin, comprising:
(I) a first polypeptide fragment, comprising:
   (a) a tag protein,
   (b) a structural region comprising a first protease cleavage site,
   (c) a short linker peptide;
(II) a second polypeptide fragment, comprising:
   (d) a first functional amino acid structural region, comprising a metal ion-dependent protease activity domain,
   (e) a structural region comprising a second protease cleavage site,
   (f) a second functional amino acid structural region, comprising a receptor-binding domain that can bind to a surface receptor of a target cell and/or a translocation domain that can mediate the transfer of the polypeptide across the vesicle membrane.

The term "tag protein" refers to a class of protein molecules that can bind to a specific ligand.

Preferably, the tag protein is selected from a tag protein known to those skilled in the art or a tag protein designed by a computer program, and is capable of specifically binding to a known substrate.

More preferably, the tag protein is selected from, but not limited to, the following proteins: a glutathione S-transferase (GSTs), C-myc, chitin binding domain, maltose binding protein (MBP), SUMO heterologous affinity moiety, monoclonal antibody or protein A, streptavidin binding protein (SBP), cellulose binding domain, calmodulin binding peptide, S tag, Strep tag II, FLA, protein A, protein G, histidine affinity tag (HAT) or polyhistidine.

Preferably, the "tag protein" can increase the solubility of the toxin polypeptide precursor in a host.

Preferably, the "tag protein" allows the presence of the toxin polypeptide precursor in a host cell in a soluble manner.

In a specific embodiment, the tag protein is located at an N terminus of the single-chain polypeptide.

In a specific embodiment, the tag protein is a glutathione s-transferase (GSTs), and more preferably, the amino acid sequence of the glutathione s-transferase is set forth in SEQ ID NO: 2.

Preferably, neither the first protease cleavage site nor the second protease cleavage site can be cleaved by a human protease or a protease produced by the host cell expressing the single-chain polypeptide.

More preferably, the first protease cleavage site and the second protease cleavage site are identical or different.

The first protease cleavage site and the second protease cleavage site are not recognized and cleaved by the host cell when being expressed or by an endogenous protease of an organism when being used.

More preferably, the specific protease is selected from, but not limited to, one of or a combination of two of the following proteases: a non-human enterokinase, a tobacco etch virus protease, a protease derived from *Bacillus subtilis,* a protease derived from *Bacillus amyloliquefaciens,* a protease derived from rhinovirus, papain, a homologue of papain of an insect, or a homologue of papain of a crustacean.

In a specific embodiment, the protease that specifically recognizes the first protease cleavage site and the protease that specifically recognizes the second protease cleavage site are both derived from the proteases of rhinoviruses.

Preferably, the second enzyme cleavage site is embedded into, partially replaces, or completely replaces a natural loop region between a first functional peptide fragment and a second functional peptide fragment. The embedding refers to an embedding between two certain amino acids in the loop region; the partial replacement refers to that the second enzyme cleavage site replaces part of the amino acid sequence of the loop region; the complete replacement refers to that the amino acid sequence of the natural loop region is completely replaced by the second enzyme cleavage site.

More preferably, the first protease cleavage site and the second protease cleavage site are selected from, but not limited to, one of or a combination of two of the following enzyme cleavage sites: DDDDK, EXXYXQS/G, HY, YH, or LEVLFQGP.

In a specific embodiment, the first protease cleavage site and the second protease cleavage site are both LEVLFQGP.

Preferably, the short linker peptide has no more than 5 amino acids.

Preferably, the short linker peptide enables the first protease cleavage site to be more easily recognized or bound to by a protease thereof.

More preferably, the short linker peptide does not affect the function of the single-chain polypeptide.

More preferably, the short linker peptide retained at the N terminus of the second polypeptide fragment does not affect the function of the second polypeptide fragment after the cleavage of the first protease cleavage site.

More preferably, a GS part of the short linker peptide has no more than 5 amino acid residues; more preferably, the short linker peptide is selected from a glycine-serine (GS for short) short peptide, GGS, GGGS, GGGGS, GSGS, GGSGS, GSGGS, GGSGS, GGGSS, and other short linker peptides.

In a specific embodiment, the amino acid sequence of the structural region comprising the first protease cleavage site and the short linker peptide is LEVLFQGPLGS.

Preferably, the metal ion-dependent protease activity domain is a Zn²⁺ -dependent protease activity domain.

Preferably, the first functional amino acid structural region and/or the second functional amino acid structural region are/is encoded by a natural sequence and/or an artificially synthesized sequence. More preferably, the first functional amino acid structural region of the single-chain polypeptide comprises a Zn²⁺ protease activity domain of the light chain of a clostridial neurotoxin.

Preferably, a target cell of the receptor-binding domain capable of binding to an antigen on the surface of a human cell in the second functional amino acid structural region refers to a target cell with an SNARE complex. For example: a nerve cell, a pancreatic cell, or other cells with an SNARE complex.

More preferably, the target cell of the second functional amino acid structural region refers to a human nerve cell or a pancreatic cell, and the receptor-binding domain is a receptor-binding domain capable of specifically binding to the human nerve cell or the pancreatic cell.

More preferably, the receptor-binding domain of the second functional amino acid structural region is a cell surface antigen-binding domain of the heavy chain of clostridial neurotoxin, and the translocation domain of the second functional amino acid structural region that mediates the transfer of the polypeptide across the vesicle membrane is a domain that mediates the transfer of clostridial neurotoxin across the vesicle membrane.

More preferably, the clostridial neurotoxin is a botulinum neurotoxin or tetanus toxin.

More preferably, the botulinum neurotoxin is selected from any one of serotypes BoNT/A to BoNT/H and derivatives thereof known in the art.

More preferably, the clostridial neurotoxin is selected from any one of tetanus toxin or a derivative thereof. More preferably, the first functional amino acid structural region comprises part or all of the light chain of BoNT/A, BoNT/B, BoNT/C, BoNT/D, BoNT/E, BoNT/F, BoNT/G, BoNT/H or tetanus toxin.

More preferably, the second functional amino acid structural region comprises part or all of the heavy chain of BoNT/A, BoNT/B, BoNT/C, BoNT/D, BoNT/E, BoNT/F, BoNT/G, BoNT/H or tetanus toxin.

In one specific embodiment, the first functional amino acid structural region is the light chain of BoNT/A, and the second functional amino acid structural region is the heavy chain of BoNT/A.

More preferably, the first functional amino acid structural region and the second functional amino acid structural region may be from different serotypes. The first functional amino acid structural region and the second functional amino acid structural region may be any combination of the above serotypes. For example, the first functional amino acid structural region is derived from the light chain of BoNT/A and the second functional amino acid structural region is derived from the heavy chain of BoNT/B, or the first functional amino acid structural region is derived from the light chain of BoNT/A and the second functional amino acid structural region is derived from the heavy chain of BoNT/C, and the like.

More preferably, the second polypeptide fragment has an amino acid sequence comprising a fragment set forth in SEQ ID NO: 7.

In a specific embodiment, the single-chain polypeptide sequentially comprises, from the N terminus, the glutathione S-transferase, LEVLFQGPLGS, the light chain of BoNT/A, LEVLFQGP, and the heavy chain of BoNT/A.

More preferably, the single-chain polypeptide has an amino acid sequence set forth in SEQ ID NO: 11.

In a second aspect of the present invention, provides a nucleic acid molecule encoding the single-chain polypeptide described above.

Preferably, the nucleic acid molecule consists of a nucleotide sequence encoding various parts of the single-chain polypeptide.

In a specific embodiment, the sequence encoding the tag protein comprises a sequence set forth in SEQ ID NO: 1.

In a specific embodiment, the nucleotide sequence encoding the structural region comprising the first protease cleavage site comprises a sequence set forth in SEQ ID NO: 3.

In a specific embodiment, the nucleotide sequence encoding the structural region comprising the second protease cleavage site comprises a sequence set forth in SEQ ID NO: 8.

Preferably, the second polypeptide fragment has an amino acid sequence comprising a sequence set forth in SEQ ID NO: 7.

Preferably, the nucleotide sequence encoding the second polypeptide fragment comprises a sequence set forth in SEQ ID NO: 6.

Preferably, the nucleotide sequence encoding the single-chain polypeptide is set forth in SEQ ID NO: 10.

In a third aspect of the present invention, provides a vector, comprising the nucleic acid molecule described above or an open reading frame encoding the single-chain polypeptide described above.

Preferably, the vector is a plasmid, a phage, a virus vector, or the like.

In a fourth aspect of the present invention, provides a cell, comprising the vector described above or expressing the single-chain polypeptide described above.

Preferably, the cell is a eukaryotic cell or a prokaryotic cell.

More preferably, the cell is selected from *Escherichia coli,* a yeast, cyanobacterium or a mammalian cell, an insect cell, a plant cell, or an amphibian cell.

More preferably, the cell is *Escherichia coli.*

In a fifth aspect of the present invention, provides a method for preparing the single-chain polypeptide described above, comprising preparing the single-chain polypeptide using any one of the nucleic acid, the vector or the cell described above.

Further, the method comprises: (1) designing a nucleic acid molecule encoding the single-chain polypeptide; (2) constructing a vector comprising the nucleic acid molecule; (3) transforming the nucleic acid vector into a suitable host cell; (4) culturing the host cell, and allowing or inducing the host cell to express the single-chain polypeptide encoded by the nucleic acid vector. Preferably, the method further comprises: (5) lysing the cell expressing the single-chain polypeptide to obtain a cell lysate comprising the single-chain polypeptide.

In a sixth aspect of the present invention, provides a single-chain polypeptide derivative, comprising:
(I) a short linker peptide;
(II) a second polypeptide fragment, comprising:
   (a) a first functional amino acid structural region, comprising a metal ion-dependent protease activity domain;
   (b) a structural region comprising a second protease cleavage site;
   (c) a second functional amino acid structural region, comprising a receptor-binding domain that can bind to a surface receptor of a target cell and/or a translocation domain that can mediate the transfer of the polypeptide across the vesicle membrane.

Preferably, the metal ion-dependent protease activity domain is a Zn²⁺ -dependent protease activity domain.

Preferably, the target cell is a human nerve cell, a pancreatic cell, or other cells with an SNARE complex.

Preferably, the single-chain polypeptide derivative is a product after the first protease cleavage site of the single-chain polypeptide described above is cleaved.

Preferably, the short linker peptide has a length of no more than 5 amino acids.

In a seventh aspect of the present invention, provides a method for preparing the single-chain polypeptide derivative described above, comprising cleaving any one of the single-chain polypeptides described above at the first protease cleavage site with the first protease to obtain the single-chain polypeptide derivative.

In an eighth aspect of the present invention, provides a method for preparing a toxin polypeptide, comprising cleaving the single-chain polypeptide described above with the first protease to remove the tag protein, and forming at least one dimer with the first functional amino acid structural region and the second functional amino acid structural region after cleaving with the second protease.

Preferably, the first functional amino acid structural region and the second functional amino acid structural region are connected into a dimeric structure through a disulfide bond.

In a ninth aspect of the present invention, provides a toxin polypeptide, comprising:
(a) a first functional amino acid structural region, comprising a metal ion-dependent protease activity domain;
(b) a second functional amino acid structural region, comprising a receptor-binding domain that can bind to a surface receptor of a target cell and/or a translocation domain that can mediate the transfer of the polypeptide across the vesicle membrane.
(a) and (b) are not connected by a peptide bond, and are preferably connected by a disulfide bond.

Preferably, a dimeric structure is formed between (a) and (b) of the toxin polypeptide.

Preferably, an amino acid residue after cleaving with the second protease is included between (a) and (b) of the toxin polypeptide.

Preferably, the toxin polypeptide has an amino acid sequence comprising a sequence set forth in SEQ ID NO: 7.

Preferably, the toxin polypeptide is prepared by the preparation method for the toxin polypeptide described above.

Preferably, the toxin polypeptide precursor described above (including the single-chain polypeptide or the single-chain polypeptide derivative) has low toxicity relative to the toxin polypeptide which is highly toxic after activation. Further preferably, the low toxicity is relative to the high toxicity after the activation. In a specific embodiment, the activity of the toxin polypeptide after the activation is at least 5000 folds higher than that of the toxin polypeptide precursor, and still further, the activity of the toxin polypeptide after the activation is 6000, 7000, 8000, 9000, 10000, 12000, 15000 or more folds higher than that of the toxin polypeptide precursor.

In a tenth aspect of the present invention, provides a composition, comprising the modified single-chain polypeptide, the single-chain polypeptide derivative or the toxin polypeptide described above.

Preferably, the composition further comprises an excipient or diluent. Further preferably, the composition is substantially free of trypsin.

Preferably, the composition is a pharmaceutical composition.

In an eleventh aspect of the present invention, provides use of the single-chain polypeptide, the single-chain polypeptide derivative, or the toxin polypeptide described above in preparing a composition.

Preferably, the composition is a pharmaceutical composition for treating a related disease by interfering with neurotransmitter release; preferably, the disease includes a neuromuscular disease, an autonomic disorder or pain, symptoms of the disease include spasmodic vocalization disorder, spasmodic torticollis, laryngeal dystonia, oromandibular dysphonia, tongue dystonia, cervical dystonia, focal dystonia, blepharospasm, strabismus, hemifacial spasm, eyelid disorder, cerebral palsy, focal spasm and other language disorders, spastic colitis, neurogenic bladder, anismus, limb spasm, tics, tremor, bruxism, anal fissure, achalasia, dysphagia and other dystonias as well as other disorders characterized by muscle group involuntary movement, lacrimation, hyperhidrosis, excessive salivation, excessive gastrointestinal secretion, secretory disorders, pain due to muscle spasm, headache and a skin disorder.

In a twelfth aspect of the present invention, provides non-therapeutic use of the single-chain polypeptide, the single-chain polypeptide derivative or the toxin polypeptide described above in ameliorating a related condition by interfering with neurotransmitter release. Preferably, the use is a cosmetic use, such as reducing facial wrinkles, shrinking facial muscles to achieve a face slimming effect, and the like.

The present invention has the following beneficial effects:
The single-chain polypeptide provided by the present invention, by adding the short linker peptide, enables the tag protein to be easier to cut, so that the single-chain polypeptide has high purity while having high yield.

The single-chain polypeptide related to the present invention has the tag protein that enables the protease structural domain of the first functional amino acid structural region not to play the function of the protease, and reduces the molecular activity (neurotoxicity) of the single-chain polypeptide to be less than ten thousandth of that of a natural toxin by replacing the natural enzyme cleavage site between the first functional amino acid structural region and the second functional amino acid structural region by a site which can be recognized only by a specific enzyme, thereby dividing the step of producing an activated molecule into the production of the single-chain polypeptide and the activation of the single-chain polypeptide. The activation of the single-chain polypeptide refers to contacting the single-chain polypeptide with a specific protease and cleavage the peptide bond between two functional peptide fragments to enable the single-chain polypeptide to be an activated molecule. In the whole production process, the production of the single-chain polypeptide occupies most of the production process, but the toxicity of the single-chain polypeptide is greatly reduced, so that the danger in the production process is reduced, and the whole industrial production is easier to carry out.

The tag protein described in the present invention refers to a heterologous affinity molecule which can bind to a specific ligand and then be separated from the ligand, so that the molecule co-expressed with the tag protein can be selected from different polypeptides or protein products more easily.

The single-chain polypeptide produced by the present invention has a target-specific binding region that may be altered or an altered active region or both, compared to the naturally occurring neurotoxin.

Compared with the prior art, the modified single-chain polypeptide of a neurotoxin capable of being activated provided by the present invention is easier to produce and purify due to higher yield, higher purity and higher safety, and can be used for preparing a properly folded and high-purity toxin polypeptide on a large scale for clinical use.

### BRIEF DESCRIPTION OF THE DRAWINGS

FIG. 1 illustrates the SDS-PAGE results of the preliminary purification of GSTs-BoNT/A and the further removal of GSTs tags, wherein lane 1 is the GSTs-BoNT/A obtained in the preliminary purification by a GSTs affinity chromatographic column, and lane 2 is BoNT/A without GSTs obtained by removing the GSTs tag protein through the digestion of the preliminarily purified protein with Rinovirus 3C Protease.
FIG. 2 illustrates the SDS-PAGE results of a high-purity BoNT/A protein obtained by further purifying the product without GSTs tag through an ion exchange column.
FIG. 3 illustrates the verification results of the dissociation of double chains of BoNT/A under reducing conditions.

### DETAILED DESCRIPTION

The present invention is further described with reference to the following specific examples, and the advantages and features of the present invention will be clearer as the description proceeds. These examples are illustrative only and do not limit the scope of the present invention in any way. It should be appreciated by those skilled in the art that modifications and replacements can be made to the details and form of the technical solutions of the present invention without departing from the spirit and scope of the present invention and that all these modifications and replacements fall within the scope of the present invention.

In the following examples, the main equipment and materials were obtained from several companies indicated below:
5-liter fermenter (customized by Shanghai Bailun), glutathione purified resin (L00206) (Genscript Biotechnology Co., Ltd.), and 3C enzyme (Z03092) (Genscript Biotechnology Co., Ltd.).

### Example 1: Design and Construction of Nucleic Acid Molecule Encoding Modified Single-Chain Polypeptide of Neurotoxin

1. the nucleic acid molecule comprises sequentially from the 5' end:
   (a) a nucleotide sequence encoding a glutathione S-transferase is set forth in SEQ ID NO: 1, the glutathione S-transferase encoded by the nucleotide sequence has an amino acid sequence set forth in SEQ ID NO: 2;
   (b) a nucleotide sequence encoding a first protease cleavage site is set forth in SEQ ID NO: 3, the amino acid sequence encoded by the nucleotide sequence is set forth in SEQ ID NO: 4;
   (c) a nucleotide sequence encoding a GS short linker peptide is set forth in SEQ ID NO: 5, which is GGATCC;
   (d) a nucleotide sequence encoding a second polypeptide fragment comprising nucleotide sequences of the light chain of BoNT/A, a second protease cleavage site, and the heavy chain of BoNT/A, is set forth in SEQ ID NO: 6, the amino acid sequence encoded by the nucleotide sequence is set forth in SEQ ID NO: 7; wherein the nucleotide sequence encoding the second protease cleavage site is set forth in SEQ ID NO: 8, the amino acid sequence encoded by the nucleotide sequence is set forth in SEQ ID NO: 9.

There can also be no natural loop region between the first functional amino acid structural region and the second functional amino acid structural region. For example, the KTKSLDKGYNK linker sequence between the light chain and the heavy chain may be removed to reduce non-specific protease cleavage.

The nucleotide sequence encoding a single-chain polypeptide is set forth in SEQ ID NO: 10, the single-chain polypeptide encoded by the nucleotide sequence has an amino acid sequence set forth in SEQ ID NO: 11.

### Example 2: Construction of Plasmid Comprising Nucleic Acid Molecule in Example 1

The genetically optimized GSTs-BoNT/A was artificially synthesized in Example 1, and NdeI and NotI enzyme cleavage sites were synthesized and added at the two ends thereof. The GSTs-BoNT/A was digested with the NdeI and NotI at 37 °C (New England Biolabs), purified by using a QIquick gel extraction kit (Qiagen), and inserted into NdeI and NotI sites in a pET28a (Novagen) plasmid vector by using a T4 DNA ligase (NEB).

### Example 3: Transfection of Plasmid Constructed in Example 2 into Host Cell

1. Preparation of competent cells: A tube of *E.coli* BL21 DE3 cells (New England Biolabs) was inoculated into a test tube containing 3 mL of LB culture medium and cultured with shaking at 37 °C overnight. On the next day, 0.5 mL of the bacterial culture was inoculated into a 250-mL flask containing 50 mL of LB culture medium and cultured with vigorous shaking (250 rpm) at 37 °C for about 3-5 h. When the OD value of the bacterial colony reached 0.3-0.4 at 600 nm, the flask was taken out and placed on ice for 10-15 min. The bacterial culture was poured into a 50 mL centrifuge tube and centrifuged at 1000 g at 4 °C for 10 min under an aseptic condition. The supernatant was discarded, and the cells were collected. 10 mL of 0.1 M CaCl₂ was added into the centrifuge tube, and the mixture was mixed well with shaking to resuspend the bacteria cells. The cells were then subjected to an ice bath for 30 min, and centrifuged at 1000 g at 4 °C for 10 min. The supernatant was discarded, and 4 mL of 0.1 M CaCl₂ pre-cooled with ice was added to resuspend the collected bacterial cells. The cells were aliquoted at 0.2 mL per tube and stored for later use within 24 h, and the remaining samples were stored in a low-temperature freezer at -70 °C.
2. Transfection: An appropriate amount of DNA (ng) and the competent *E. coli* were mixed, placed in an ice bath for 15 min, heat-shocked at 42 °C for 30 s, placed in the ice bath for 5 min, shaken at 250 rpm for 1 h in an SOC medium, smeared on a plate with antibiotics, and cultured at 37 °C overnight.

### Example 4: Culture of Host Cell and Induction of Expression of Single-Chain Polypeptide

The composition and ratio of the culture medium: 11.8g/L of tryptone, 23.6 g/L of yeast extract, 9.4 g/L of K₂HPO₄, 2.2 g/L of KH₂PO₄, and 4 mL/L of glycerol.

The culture condition: The cells were cultured with shaking at 250 rpm at 37 °C overnight.

Inducing expression: l mM IPTG was added to induce expression at 25 °C for 5 h when the *E*. *coli* grew to a stage in which the OD₆₀₀ = 1. The OD₆₀₀ threshold may be 0.2-1.5, the temperature threshold may be 37 °C to 10 °C, and the expression time may be 5-16 h.

Harvesting cells: *E. coli* was collected by centrifugation at 3000 rpm for 30 min.

### Example 5: Identification of Expression Level of Single-Chain Polypeptide

GSTs-BoNT/A: A glutathione purification resin chromatographic column having a diameter of 1 cm and a height of 1 cm was prepared using Genscript glutathione purification resin, 25 mL of the lysate was slowly poured to the upper layer of the chromatographic column (be careful not to flush the resin), and the lysate was slowly loaded on the column to adsorb GSTs-BoNT/A in the lysate for one hour. The GSTs-BoNT/A was eluted according to a conventional method.

The conventional method is as follows: A chromatographic column was washed with 20 column volumes of a phosphate buffer. GSTs-BoNT/A elution was eluted with 10 column volumes of a freshly prepared 10 mM glutathione elution buffer (0.154 g reduced glutathione dissolved in 50 mL of 50 mM Tris-HCl (pH 8.0)). The elution of the fusion protein was monitored by absorbance reading at 280 nm. GSTs tag protein of the eluted protein was cleaved under the effect of a Rinovirus 3C Protease.

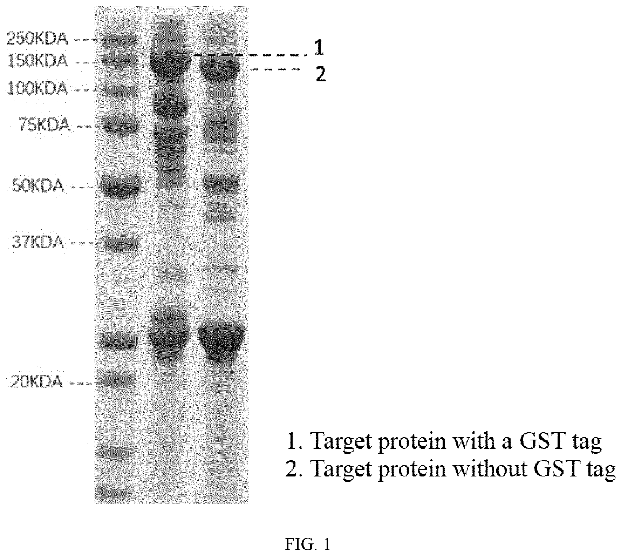

Products before and after the removal of the GSTs tag protein were subjected to a conventional SDS-PAGE experiment shown in FIG. 1. Compared with lane 1 in which the tag protein was not cleaved, the GSTs tag protein of lane 1 was cleaved to obtain a BoNT/A molecule without GSTs under the effect of Rinovirus 3C Protease.

Determination of the proportion of GSTs-BoNT/A in the total protein level: The purified GSTs-BoNT/A was electrophoretically separated at 200 volts by using 4-12% SDS-PAGE (Biorad) and a major band with a molecular weight of 175 kd was GSTs-BoNT/A.

### Example 6: Removal of Tag Protein and Purification

The product in Example 5 was subjected to removal of GSTs tag protein and purification of toxic polypeptides:
The GSTs-BoNT/A re-adsorbed on a glutathione purification resin chromatographic column was treated using Genscript 3C enzyme. The first enzyme cleavage site between the GSTs and BoNT/A was cleaved under the effect of the 3C enzyme. GSTs were separated. The second enzyme cleavage site between the light chain and the heavy chain of BoNT/A was cleaved.

The glutathione purification resin chromatographic column was treated with a phosphate buffer. The GSTs tag protein was retained on the column and was thus removed, while the light chain and the heavy chain of BoNT/A were eluted by the phosphate buffer.

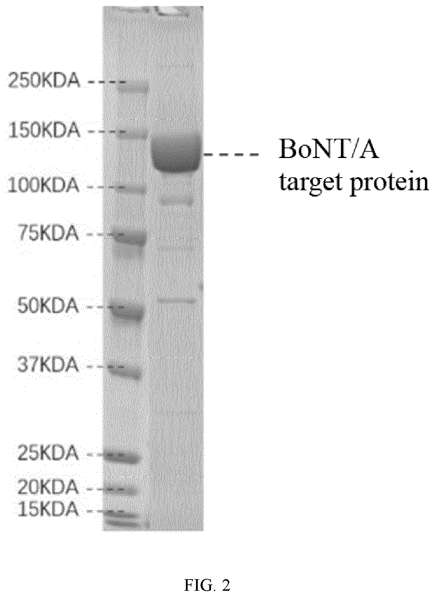

A product after the removal of GSTs was subjected to a conventional SDS-PAGE experiment shown in FIG. 2, and the obtained bands did not include the GSTs tag protein, indicating that the GSTs tag protein had been completely removed. The SDS-PAGE results were scanned, and the purity of the obtained BoNT/A was above 90% by calculation based on the grey density of the band.

GSS, GSGS and GGSGS polypeptides were adopted to replace a GS part of a short linker peptide, which demonstrated a similar effect to that of GS. The tag protein can be well exposed and thus completely cleaved.

### Example 7: Dissociation of Dimer BoNT/A Double Chains under Reducing Conditions

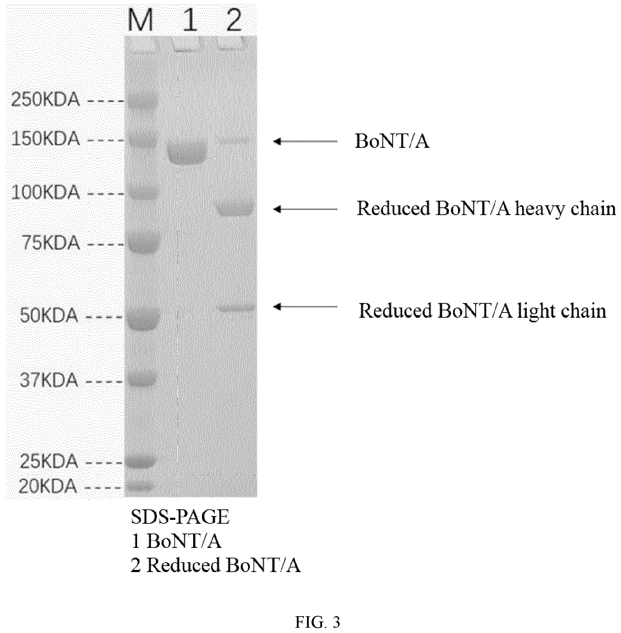

The products of Example 6 were subjected to a reduction experiment:
A sample was treated by using 100 mM dithiothreitol at 100 °C for 5 min to reduce the sample, and electrophoretically separated at 200 volts by the 4-12% SDS-PAGE (Biorad) to separate the heavy chain and the light chain. As shown in FIG. 3, the products obtained in Example 6 were subjected to reduction under reducing conditions, and the obtained products were subjected to a conventional SDS-PAGE experiment to obtain two different bands with molecular weights of 100 KDa and 50 KDa, respectively, which demonstrated that the product formed in Example 6 was a dimeric structure in which two peptide fragments were linked by a disulfide bond.

### Example 8: Toxicity Test of GSTs-BoNT/A

The GSTs-BoNT/A obtained in Example 5 had an LD₅₀ of 45-450 ng after intraperitoneal administration in mice; considering the purity of the injected botulinum toxin protein, the converted LD₅₀ was 22.5-225 ng, with a mid-value of 123.75 ng. The BoNT/A obtained in Example 5 had an LD₅₀ of 0.02-0.05 ng after intraperitoneal administration in mice. Considering the purity of the injected botulinum toxin protein, the converted LD₅₀ was 0.006-0.015 ng, with a mid-value of 0.0105 ng (see Table 1).

The GSTs-BoNT/A had the activity of botulinum toxin, and the median lethal dose (LD₅₀) thereof was approximately 11786 times higher than the LD₅₀ of the BoNT/A protein after intraperitoneal administration in mice, which indicates that the activity of a toxin polypeptide precursor molecule of GSTs-BoNT/A recombinant protein is approximately 11786 times weaker than that of the final product BoNT/A. The experiment demonstrates that the toxin polypeptide precursor molecule of the GSTs-BoNT/A recombinant protein has the activity of botulinum toxin. Due to the ultra-high toxicity of botulinum toxin, high safety operation precautions are required in the manufacturing process before the activation treatment of a precursor molecule.

**Table 1. comparison of GSTs-BoNT/A and BoNT/A molecules in mice biotoxicity study**

| Test sample | Description | Dose (ng) | Lethality rate of mice 72 h after administration (%) |
|---|---|---|---|
| GST-BoNT/A (50% purity) | GST-BoNT/A (50% purity) | 450 | 100 |
| | | 45 | 50 |
| | | 4.5 | 0 |
| | | 0.45 | 0 |
| BoNT/A (30% purity) | BoNT/A (30% purity) | 0.05 | 100 |
| | | 0.02 | 0 |
| | | 0.01 | 0 |
| | | 0.005 | 0 |

The present invention exemplifies the construction of a nucleic acid molecule comprising GSTs, a first protease cleavage site, a short linker peptide, and a light chain of BoNT/A, a second protease cleavage site, and a heavy chain of BoNT/A; further constructs a plasmid containing the nucleic acid molecule and capable of expressing *GSTs-BoNT*/*A* in *E. coli; E.coli* transfected with the plasmid expresses single-chain GSTs-BoNT/A, the single-chain polypeptide is proved to be less than ten thousandth of toxicity of the activated final product BoNT/A. Protein purification and GSTs elution prove that due to the addition of the short linker peptide, enzyme cleavage sites are easier to identify and cleave by a protease than the enzyme cleavage sites without a short linker peptide. Since the first protease cleavage site and the second protease cleavage site are identical, when GSTs are removed, the second protease cleavage site is cleaved, and the peptide bond between the first functional amino acid structural region and the second functional amino acid structural region is cleaved and the two structural regions are linked by a disulfide bond to form active BoNT/A, which is proved to have toxicity equivalent to that of a natural product.

The preferred embodiments of the present invention are described in detail above, which, however, are not intended to limit the present invention. Within the scope of the technical concept of the present invention, various simple modifications can be made to the technical solution of the present invention, all of which will fall within the protection scope of the present invention.

In addition, it should be noted that the various specific technical features described in the above specific embodiments can be combined in any suitable manner where the features do not contradict each other. In order to avoid unnecessary repetition, such combinations will not be illustrated separately.

## Claims

1. A modified single-chain polypeptide of a neurotoxin, comprising:
(I) a first polypeptide fragment, comprising:
(a) a tag protein,
(b) a structural region comprising a first protease cleavage site,
(c) a short linker peptide;
(II) a second polypeptide fragment, comprising:
(d) a first functional amino acid structural region, comprising a metal ion-dependent protease activity domain;
(e) a structural region comprising a second protease cleavage site;
(f) a second functional amino acid structural region, comprising a receptor-binding domain that can bind to a surface receptor of a target cell and/or a translocation domain that can mediate the transfer of the polypeptide across the vesicle membrane.

2. The single-chain polypeptide according to claim 1, wherein the first protease cleavage site and the second protease cleavage site are identical.

3. The single-chain polypeptide according to claim 1 or 2, wherein the short linker peptide has no more than 5 amino acid residues.

4. The single-chain polypeptide according to any one of claims 1-3, wherein the first protease cleavage site and the second protease cleavage site are not recognized and cleaved by a host cell when expressed or by an endogenous protease of a body when used; preferably, the first protease cleavage site and the second protease cleavage site are selected from any one of the following protease recognition and cleavage sites: a non-human enterokinase, a tobacco etch virus protease, a protease derived from *Bacillus subtilis,* a protease derived from *Bacillus amyloliquefaciens,* a protease derived from rhinovirus, a papain, a homologue of papain of an insect, or a homologue of papain of a crustacean.

5. The single-chain polypeptide according to any one of claims 1-4, wherein the first functional amino acid structural region is derived from a light chain of the neurotoxin or comprises at least a Zn²⁺ protease activity domain of the light chain of the neurotoxin; the second functional amino acid structural region is derived from a heavy chain of the neurotoxin or comprises at least a receptor-binding domain of the heavy chain of the neurotoxin and/or a translocation domain mediating a transfer of the polypeptide across a vesicle membrane.

6. A nucleic acid molecule encoding the single-chain polypeptide according to any one of claims 1-5.

7. A vector, comprising the nucleic acid molecule according to claim 6 or an open reading frame encoding the single-chain polypeptide according to any one of claims 1-5.

8. The vector according to claim 7, wherein the vector is a plasmid.

9. A cell, comprising the vector according to any one of claims 7-8 or expressing the single-chain polypeptide according to any one of claims 1-5.

10. The cell according to claim 9, wherein the cell is selected from any prokaryotic or eukaryotic cell; preferably, the cell is selected from *Escherichia coli,* yeast, cyanobacteria or mammalian cell lines.

11. A method for preparing the single-chain polypeptide according to any one of claims 1-5, comprising preparing the single-chain polypeptide using the nucleic acid molecule according to claim 6, the vector according to any one of claims 7-8 or the cell according to any one of claims 9-10.

12. A single-chain polypeptide derivative, comprising:
(I) a short linker peptide; and
(II) a second polypeptide fragment, comprising:
(a) a first functional amino acid structural region, comprising a metal ion-dependent protease activity domain;
(b) a structural region comprising a second protease cleavage site;
(c) a second functional amino acid structural region, comprising a receptor-binding domain that can bind to a surface receptor of a target cell and/or a translocation domain that can mediate the transfer of the polypeptide across the vesicle membrane.

13. A method for preparing the single-chain polypeptide derivative according to claim 12, comprising cleaving the single-chain polypeptide according to any one of claims 1-5 at the first protease cleavage site with the first protease to obtain the single-chain polypeptide derivative.

14. A method for preparing a toxin polypeptide, comprising cleaving the single-chain polypeptide according to any one of claims 1-5 with the first protease to remove the tag protein, and forming at least one dimeric structure with the first functional amino acid structural region and the second functional amino acid structural region after cleaving with the second protease.

15. A toxin polypeptide prepared by the method according to claim 14.

16. A composition, comprising the single-chain polypeptide according to any one of claims 1-5, the single-chain polypeptide derivative according to claim 12 or the toxin polypeptide according to claim 15.

17. The composition according to claim 16, wherein the composition is a pharmaceutical composition; preferably, the pharmaceutical composition further comprises an excipient or diluent.

18. Use of the single-chain polypeptide according to any one of claims 1-5, the single-chain polypeptide derivative according to claim 12 or the toxin polypeptide according to claim 15 in preparing a composition.

19. The use according to claim 18, wherein the composition is a pharmaceutical composition for treating a related disease by interfering with neurotransmitter release; preferably, the disease includes a neuromuscular disease, symptoms of the disease include one or more of spasmodic vocalization disorder, spasmodic torticollis, laryngeal dystonia, oromandibular dysphonia, tongue dystonia, cervical dystonia, focal dystonia, blepharospasm, strabismus, hemifacial spasm, eyelid disorder, cerebral palsy, focal spasm and other language disorders, spastic colitis, neurogenic bladder, anismus, limb spasm, tics, tremor, bruxism, anal fissure, achalasia, dysphagia and other dystonias as well as other disorders **characterized by** muscle group involuntary movement, lacrimation, hyperhidrosis, excessive salivation, excessive gastrointestinal secretion, secretory disorders, pain due to muscle spasm, headache or a skin disorder.

20. Non-therapeutic use of the single-chain polypeptide according to any one of claims 1-5, the single-chain polypeptide derivative according to claim 12 or the toxin polypeptide according to claim 15 in ameliorating a related condition by interfering with neurotransmitter release, wherein the use is preferably a cosmetic use.
